(19) **Europäisches Patentamt / European Patent Office / Office européen des brevets**

(11) **EP 1 328 474 B1**

(12) **FASCICULE DE BREVET EUROPEEN**

(45) Date de publication et mention
de la délivrance du brevet:
**11.04.2007 Bulletin 2007/15**

(51) Int Cl.:
***C01B 33/193*** *(2006.01)*  ***A61K 8/25*** *(2006.01)*
***A61Q 11/00*** *(2006.01)*

(21) Numéro de dépôt: **01978514.6**

(86) Numéro de dépôt international:
**PCT/FR2001/003104**

(22) Date de dépôt: **09.10.2001**

(87) Numéro de publication internationale:
**WO 2002/030817 (18.04.2002 Gazette 2002/16)**

(54) **GRANULES DE SILICE DE HAUTE STRUCTURE ET LEUR UTILISATION DANS LES COMPOSITIONS DENTAIRES**

HOCHSTRUKTURIERTE KIESELSÄUREGRANULATE UND IHRE VERWENDUNG FÜR DENTALE ZUSAMMENSETZUNGEN

HIGH-STRUCTURED SILICA GRANULES AND THEIR USE IN DENTAL COMPOSITIONS

(84) Etats contractants désignés:
**AT BE CH CY DE DK ES FI FR GB GR IE IT LI LU MC NL PT SE TR**

(30) Priorité: **12.10.2000 FR 0013085**

(43) Date de publication de la demande:
**23.07.2003 Bulletin 2003/30**

(73) Titulaire: **RHODIA CHIMIE**
**92512 Boulogne Billancourt Cedex (FR)**

(72) Inventeur: **Kiefer, Jean-Claude**
**F-60460 Les Précis-Précy-sur-seine (FR)**

(74) Mandataire: **Delenne, Marc**
**Rhodia Services,**
**Direction de la Propriété Industrielle,**
**40, rue de la Haie-Coq**
**93306 Aubervilliers Cedex (FR)**

(56) Documents cités:
**EP-A- 0 268 763**     **WO-A-96/09033**
**WO-A-96/09034**     **WO-A-97/30126**
**WO-A-99/31184**     **GB-A- 2 272 640**

Printed by Jouve, 75001 PARIS (FR)

**Description**

La présente invention a pour objet des granulés de silice de haute structure (épaississante) obtenus par granulation d'une silice de précipitation de haute structure (épaississante) et leur utilisation dans les compositions dentaires comme agent épaississant pour apporter des propriétés sensorielles en bouche.

Il est connu (GB-A-2 272 640) de préparer des granulés de silice de 0,03 à 3 mm par granulation, par de l'eau, de silice dite "épaississante" de particules primaires de 0,01 à 0,2 $\mu$m. Ces granulés peuvent être utilisés dans les compositions dentifrices ; ils sont friables dans les conditions de brossage. Il est proposé de granuler à l'eau de silice "épaississante" Sident22S dans un granulateur haute vitesse à double pale.

Il est connu de granuler par de l'eau des mélanges de silice de basse structure (abrasive) et de haute structure (épaississante) et d'ajuster la cohésion des agglomérats obtenus par le rapport entre les deux types de silice ; ces agglomérats sont friables au brossage et peuvent être utilisés comme agent de nettoyage apportant un bénéfice sensoriel dans les compositions dentifrices (WO 96/09033 et WO 96/09034). Le document WO 96/09033 enseigne que des agglomérats formés par granulation à l'eau dans un mélangeur Sirman CV6 de Metcalfe Catering Equipment Ltd., de silice de haute structure (épaississante) seule, ne se cassent pas pendant le temps imparti au brossage.

La Demanderesse a trouvé des granulés en silice épaississante ne contenant pas de silice abrasive et présentant une force de cohésion suffisante pour être introduits dans les compositions dentifrices, mais toutefois limitée pour être en partie cassables au brossage. Ces cogranulés peuvent être utilisés comme agent épaississant dans les compositions dentaires ou buccales, agent apportant en outre des propriétés sensorielles en bouche, à savoir la perception d'une propriété de rugosité (roughness perception) en début de brossage, puis perception d'onctuosité et d'un phénomène d'éclatement des granulés (breackdown perception) au fur et à mesure de l'avancement du brossage. Ces granulés étant de structure silice parfaitement homogène, à savoir constitués essentiellement de silice de haute structure (épaississante), peuvent être mis en oeuvre en quantité relativement importante dans les dentifrices ou les compositions dentaires, et ce sans risque d'endommager l'émail des dents, et peuvent remplacer au moins partiellement des épaississants habituels comme les gommes, les poudres de silice épaississante ...

Un premier objet de l'invention, consiste en des granulés de silice de précipitation de haute structure (épaississante) présentant un diamètre médian d'au moins 150 $\mu$m, de préférence d'au moins 250 $\mu$m, obtenus par granulation par voie humide de silice de précipitation de haute structure (épaississante), séchage et éventuellement tamisage, lesdits granulés étant caractérisés en ce qu'ils présentent un facteur de cohésion FC d'au moins 0,3 , de préférence de 0,4 à moins de 0,9 et en ce que l'étape de granulation est réalisée par mise en oeuvre

   * d'un appareil à rotor comprenant des moyens de cisaillement susceptibles de développer une vitesse périphérique en bout de rotor allant de 0,5 m.s à 8 m.s, de préférence de 2 m.s à 6 m.s
   * d'au moins une poudre de silice de précipitation de haute structure (épaississante) présentant

   • une prise d'huile DOP d'au moins 230ml/100g, de préférence d'au moins 290 ml/100 g
   • un diamètre médian $D_{50}$ d'au moins 14 $\mu$m, de préférence de 14 à 80 $\mu$m, tout particulièrement de 14 à 30 $\mu$m

   * et d'eau (W) comme liant

le rapport pondéral Rm de la quantité d'eau totale (WT) à la quantité de silice exprimée en sec étant supérieur à 1,4 et inférieur à 3, de préférence supérieur à 1,5 et inférieur à 3.

On entend par eau totale (WT) non seulement celle introduite comme liant, mais également celle incluse dans la poudre de silice sèche.

Le taux d'humidité de la poudre de silice peut être de l'ordre de 3 à 15% en poids ; ce taux est généralement de l'ordre de 6 à 12% en poids.

A titre d'exemple d'appareils de granulation pouvant être mis en oeuvre pour réaliser l'invention, on peut mentionner les granulateurs à rotor équipé de socs de charrue, tels que ceux du type LÖDIGE (de Lôdige), WAM (de Wam), DRAIS (de Drais) ... fonctionnant en continu ou discontinu.

Le temps de granulation est généralement de l'ordre de 10 à 45 minutes.

Dans un appareil fonctionnant en discontinu, le temps de séjour des matières premières dans le granulateur (incluant le temps nécessaire à l'introduction de l'eau) peut être généralement de l'ordre de 15 à 60 minutes.

Le temps de séjour ou de granulation est notamment fonction de l'appareil de granulation mis en oeuvre, de son volume, de la vitesse périphérique développée en bout de rotor, de la quantité d'eau de granulation mise en oeuvre.

La détermination de ces différents paramètres peut aisément être établie par l'homme du métier.

Ainsi, pour une vitesse périphérique développée en bout de rotor d'au plus 4 m.s, un temps de granulation de 20 à 45 minutes est conseillé ; pour un appareil fonctionnant en discontinu, cela correspond à un temps de séjour des matières premières dans le granulateur de l'ordre de 25 à 60 minutes.

Pour une vitesse périphérique développée en bout de rotor supérieure à 4 m.s, un temps de granulation de 10 à 20 minutes est conseillé; pour un appareil fonctionnant en discontinu, cela correspond à un temps de séjour des matières premières de l'ordre de 15 à 45 minutes.

Pour une vitesse périphérique développée en bout de rotor d'au plus 4 m.s, il est conseillé de mettre en oeuvre un rapport Rm de la quantité d'eau totale (WT) à la quantité de silice supérieur à 1,8 et inférieur à 2,5. Pour une vitesse périphérique développée en bout de rotor supérieure à 4 m.s, il est conseillé de mettre en oeuvre un rapport Rm supérieur à 1,4 et inférieur à 3, de préférence supérieur à 1,5 et inférieur à 3.

L'opération de granulation peut être réalisée de préférence à température ambiante (température du lieu de l'installation) en discontinu par pulvérisation de l'eau (W) sur la poudre maintenue en mouvement ou en continu par alimentation en continu et mise en contact de l'eau (W) et de la poudre de silice.

Le produit obtenu par granulation est ensuite transféré dans un appareil de séchage (étuve, lit fluidisé, lit traversé ... ou autre moyen connu de séchage), où il est séché jusqu'à ce qu'il présente une humidité résiduelle de l'ordre de 3 à 15% en poids (généralement de l'ordre de 6 à 12% en poids). La température de l'air de séchage peut être par exemple de l'ordre de 100 à 120°C.

Il peut être conseillé, pour une bonne réalisation de l'invention, de mettre en oeuvre une poudre de silice dont le diamètre $D_{10}$ est d'au moins 1 $\mu$m, de préférence d'au moins 3 $\mu$m, et inférieur à 10$\mu$m.

Les granulés obtenus peuvent ensuite, si nécessaire, être tamisés afin d'éliminer ceux en dehors de la coupe granulométrique recherchée.

D'une manière préférentielle, les granulés de l'invention présentent un facteur de cohésion FC de 0,45 à 0,75, tout particulièrement de 0;5 à 0,7. Tout préférentiellement ceux-ci présentent en outre un diamètre médian de 300 à 450 $\mu$m, tout particulièrement de 300 à 400 $\mu$m.

La prise d'huile DOP de la silice est déterminée selon la norme ISO 787/5 en mettant en oeuvre du dioctylphtalate, sur des particules de silice séchées selon le protocole décrit dans cette même norme.

La répartition granulométrique de la silice de départ est déterminée après mise en dispersion et traitement de deux minutes aux ultrasons (puissance 100%), par diffraction laser voie humide dans un appareil MALVERN MASTERSIZER (de Malvern Instruments).

Les paramètres de l'appareil sont réglés comme suit:

* lentille 300 RF (plage granulométrique de 0,05 à 880 $\mu$m)
* configuration 3-$SiO_2$ (indice de réfraction 1,4600 , 0,0100)
* modèle d'analyse : polydisperse
* pompe et agitation : 60%
* dispersant : eau

Le diamètre $D_{50}$ correspond à la taille de pas plus de 50% en volume des particules.

Le diamètre $D_{10}$ correspond à la taille de pas plus de 10% du volume des particules.

Le diamètre $D_{90}$ correspond à la taille de pas plus de 90% du volume des particules.

La répartition granulométrique des granulés finaux est effectuée avec le même appareil sur la coupe 630 pm-1 25 $\mu$m obtenue par tamisage.

La répartition granulométrique est déterminée après mise en dispersion, sans traitement aux ultrasons.

Le diamètre $D_{50}$ correspond à la taille de pas plus de 50% en volume des granulés de silice.

Le diamètre $D_{10}$ correspond à la taille de pas plus de 10% du volume des granulés de silice.

Le diamètre $D_{90}$ correspond à la taille de pas plus de 90% du volume des granulés de silice.

La répartition granulométrique est à nouveau contrôlée après avoir soumis la dispersion à 2 minutes d'ultrasons ; la puissance d'ultrasons est de 50%.

La force de cohésion FC des granulés est déterminée par le rapport du $D_{50}$ des granulés traités aux ultrasons (puissance 50%) au $D_{50}$ des granulés avant traitement aux ultrasons

$$FC = D_{50} \text{ après ultrasons} / D_{50} \text{ sans ultrasons}$$

La silice mise en oeuvre sous forme de poudre est une silice de précipitation de prise d'huile DOP d'au moins 230 ml/100 g ; elle peut être obtenue selon les techniques connues de préparation de silice de précipitation épaississante à partir d'une solution aqueuse d'un silicate de métal alcalin (par exemple un silicate de sodium ou de potassium de rapport molaire $SiO_2/M_2O$ de 2 à 4) et d'un agent d'acidification (par exemple l'acide sulfurique, nitrique, chlorhydrique, carbonique, acétique, formique ...).

Ces silices peuvent présenter une surface spécifique BET d'au moins 140 m²/g (déterminée selon la méthode de BRUNAUER - EMMET - TELLER décrite dans "The journal of the American Chemical Society", Vol. 60, page 309, février 1938 et correspondant à la norme ISO 5794/1 [annexe D]).

Parmi les silices du commerce pouvant être mises en oeuvre comme poudre de silice, on peut citer les silices connues sous les dénominations Tixosil 38AB de Rhodia, Hisil 267 de PPG, ...

Les granulés selon l'invention peuvent présenter une DOP d'au moins 190 ml/100 g, de préférence d'au moins 210 ml/100 g.

Les granulés faisant l'objet de l'invention peuvent être utilisés comme additifs dans les compositions pour le nettoyage et/ou le traitement de la peau, des dents et autres surfaces dures. Ils peuvent être mis en oeuvre à raison de 0,1 à 50% du poids desdites compositions.

Ils peuvent être par exemple mis en oeuvre comme agent exfoliant dans les compositions cosmétiques (gels douches, crèmes ...) à raison de 0,5 à 5% du poids desdites compositions, dans les compositions de nettoyage des surfaces dures (poudres ou crèmes à récurer ...) à raison de 20 à 50% du poids desdites compositions.

Les granulés préparés selon le procédé de granulation ci-dessus décrit et notamment ceux présentant un facteur de cohésion FC de 0,45 à 0,75 , tout particulièrement de 0,5 à 0,7 , sont tout à fait aptes à être utilisés comme agents épaississants à effet sensoriel en bouche pour compositions dentaires. Tout préférentiellement ceux-ci présentent en outre un diamètre médian de 300 à 450 $\mu$m, tout particulièrement de 300 à 400 $\mu$m.

Selon l'invention, lesdits granulés peuvent en outre comprendre des agents colorants, opacifiants (notamment du dioxyde de titane), des parfums, des agents actifs vis à vis des dents ou de la paroi buccale, thérapeutiques ou non thérapeutiques, comme des agents fluorés, du citrate de zinc, des agents antitartre, des agents anti-caries ....

Ces granulés peuvent être utilisés notamment comme agent épaississant dans les compositions buccales, notamment dans les compositions dentifrices (pâtes, gels notamment), apportant aux dites compositions des propriétés sensorielles en bouche.

En effet, étant de cohésion limitée, ces granulés apportent un effet sensoriel par désintégration au brossage, se traduisant par la perception d'une certaine rugosité (roughness perception, crunchy) en début de brossage, puis la perception d'un phénomène d'éclatement des granulés (breackdown perception) et d'onctuosité au fur et à mesure de l'avancement du brossage.

En outre, étant obtenus à partir de silices dites de "haute structure" ou "épaississantes" (prise d'huile DOP élevée), ils peuvent être mis en oeuvre en quantité relativement importante dans les compositions dentaires sans donner de sensation désagréable de sable ("gritty feeling") et sans risque de dommages pour l'émail des dents. Ils peuvent remplacer au moins partiellement les épaississants habituels comme les gommes ou les poudres de silice épaississante.

Lesdits granulés peuvent être mis en oeuvre dans les compositions dentaires ou buccales, à raison 0,1 à 12%, de préférence de 0,5 à 10% en poids desdites compositions.

Ces compositions peuvent en outre renfermer d'autres ingrédients habituels, en particulier des agents abrasifs minéraux, insolubles dans l'eau, des agents épaississants, des humectants...

Comme agents abrasifs, on peut mentionner en particulier les silices abrasives, le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50% du poids de la composition dentaire.

Parmi les agents épaississants on peut mentionner tout particulièrement les silices épaississantes en quantité pouvant aller jusqu'à 15% du poids de ladite composition, la gomme xanthane, la gomme guar, les carraghénanes, les dérivés de la cellulose, les alginates, en quantité pouvant aller jusqu'à 5% du poids de ladite composition...

Parmi les agents humectants on peut citer par exemple le glycérol, le sorbitol, les polyéthylèneglycols, les polypropylèneglycols, le xylitol, en quantité de l'ordre de 2 à 85%, de préférence de l'ordre de 3 à 55% du poids de composition dentifrice exprimé en sec.

Ces compositions dentifrices peuvent en outre comporter des agents tensioactifs, des agents détergents, des colorants, des antibactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym...)...

La présente invention a également pour objet l'utilisation des granulés en silice de haute structure (épaississante) ci-dessus, notamment ceux présentant une force de cohésion FC de l'ordre de 0,45 à 0,75 , de préférence de 0,5 à 0,7 ,

comme agent épaississant à effet sensoriel en bouche dans les compositions dentifrices. Lesdits granulés peuvent être présents à raison de 0,1 à 12%, de préférence de 0,5 à 10% du poids desdites compositions.

Ces compositions peuvent se présenter sous la forme d'une pâte, d'un gel ...

Un dernier objet de l'invention consiste en des compositions dentaires, comprenant lesdits granulés en silice de haute structure (épaississante) ci-dessus, notamment ceux présentant une force de cohésion FC de l'ordre de 0,45 à 0,75 , de préférence de 0,5 à 0,7 ; Lesdits granulés peuvent être présents à raison de 0,1 à 12%, de préférence de 0,5 à 10% du poids desdites compositions.

Les exemples suivants sont donnés à titre illustratif.

### Exemple 1

Matériel utilisé

- * pompe volumétrique MOINEAU
- * granulateur LÖDIGE de 20 litres
- * buse pression LECHLER (Ref. : 460.403.17 $\varnothing$ 0,85 mm)
- * cuve d'alimentation en verre de 8 litres avec agitation
- * balance IR pour les essais secs (160°C)

Poudre de silice : Tixosil 38AB de Rhodia (DOP de 330 ml/100 g)

. distribution granulométrique
(mesure au MALVERN 100% US)

|          | sans ultrasons | après ultrasons |
|----------|----------------|-----------------|
| $D_{10}$ | 8,43 $\mu$m    | 7,52 $\mu$m     |
| $D_{50}$ | 24,65 $\mu$m   | 18,72 $\mu$m    |
| $D_{90}$ | 102,50 $\mu$m  | 66,25 $\mu$m    |

. humidité = 10%

Granulation

1566 g de poudre de silice sont introduits dans le granulateur.
La vitesse d'agitation du granulateur Lödige est réglée à 200 tours/minute (vitesse périphérique de 2,6 m.s.).
3132 g d'eau sont introduits dans la cuve d'alimentation du Lôdige, ce qui correspond à un rapport (Rm) de la quantité d'eau totale (WT) à la quantité totale (ST) de silice, (Rm) = (WT)/(ST), de 2.
Le potentiomètre de la pompe est réglé à 5.
L'eau est ensuite pulvérisée sur la poudre de silice pendant 4minutes et 30 secondes ; la pression de pulvérisation est inférieure à 1 bar.
Après arrêt de la pompe à la fin de la pulvérisation, le malaxage (temps de granulation TG) est encore poursuivi pendant 15 minutes et 30 secondes.
Le produit obtenu est vidangé dans un bac ; son extrait sec est de 31,8% en poids. Il est ensuite séché en étuve à 90-100°C pendant une nuit.

Analyse granulométrique du produit obtenu

* tamisage

|                         |     |
|-------------------------|-----|
| >0,630 mm               | 40% |
| [0,125 mm ; 0,630 mm]   | 53% |
| <0,250 mm               | 7%  |

La coupe [0,125 mm ; 0,630 mm] est conservée.
* humidité : 6%
* résultats

| diamètre des granulés | sans ultrasons en μm | après 2 minutes d'ultrasons en μm |
|:---:|:---:|:---:|
| Dio | 179 | 23 |
| $D_{50}$ | 371 | 219 |
| $D_{90}$ | 647 | 550 |

ce qui correspond à un facteur de cohésion FC de 0,59.

## Exemple 2

Matériel utilisé

  * pompe volumétrique MOINEAU
  * granulateur WAM de 150 litres
  * buse pression LECHLER (Ref. : 460.403.17 ; ∅ 0,85 mm)
  * cuve d'alimentation en verre de 30 litres avec agitation
  * balance IR pour les essais secs (160°C)

Poudre de silice : Tixosil 38AB de Rhodia (DOP de 330 ml/100g)

. distribution granulométrique
(mesure au MALVERN 100% US)

|  | sans ultrasons | après ultrasons |
|---|---|---|
| $D_{10}$ | 8,43 μm | 7,52 μm |
| $D_{50}$ | 24,65 μm | 18,72 μm |
| $D_{90}$ | 102,50 μm | 66,25 μm |

. humidité = 10%

Granulation

12 kg de poudre de silice sont introduits dans le granulateur.
La vitesse d'agitation du granulateur WAM est réglée à 210 tours/minute (vitesse périphérique de 5,2 m.s.).
21,6 kg d'eau sont introduits dans la cuve d'alimentation du WAM, ce qui correspond à un rapport (Rm) de la quantité d'eau totale (WT) à la quantité totale (ST) de silice, (Rm) = (WT)/(ST), de 1,8.
Le potentiomètre de la pompe est réglé à 5.
L'eau est ensuite pulvérisée sur la poudre de silice pendant 4 minutes ; la pression de pulvérisation est inférieure à 1 bar.
Après arrêt de la pompe à la fin de la pulvérisation, le malaxage (temps de granulation TG) est encore poursuivi pendant 15 minutes.
Le produit obtenu est vidangé dans un bac ; son extrait sec est de 33,5% en poids. Il est ensuite séché en étuve à 90-100°C pendant une nuit.

Analyse granulométrique du produit obtenu

* tamisage

| | |
|---|---|
| >0,630 mm | 17% |
| [0,125 mm ; 0,630 mm] | 58% |
| <0,250 mm | 25% |

La coupe [0,125 mm ; 0,630 mm] est conservée.

* humidité : 4,7%

* résultats

| diamètre des granulés | sans ultrasons en $\mu$m | après 2 minutes d'ultrasons en $\mu$m |
|---|---|---|
| $D_{50}$ | 364 | 178 |

ce qui correspond à un facteur de cohésion FC de 0,49.

**Revendications**

1. Granulés de silice de précipitation de haute structure (épaississante) présentant un diamètre médian d'au moins 150 $\mu$m, de préférence d'au moins 250 $\mu$m, obtenus par granulation par voie humide de silice de précipitation de haute structure (épaississante), séchage et éventuellement tamisage,
   lesdits granulés étant **caractérisés en ce qu'**ils présentent un facteur de cohésion FC d'au moins 0,3 , de préférence de 0,4 à moins de 0,9 et **en ce que** l'étape de granulation est réalisée par mise en oeuvre

   * d'un appareil à rotor comprenant des moyens de cisaillement susceptibles de développer une vitesse périphérique en bout de rotor allant de 0,5 m.s à 8 m.s, de préférence de 2 m.s à 6 m.s
   * d'au moins une poudre de silice de précipitation de haute structure (épaississante) présentant

      . une prise d'huile DOP d'au moins 230 ml/100 g, de préférence d'au moins 290 ml/100 g
      . un diamètre médian $D_{50}$ d'au moins 14 $\mu$m, de préférence de 14 à 80 $\mu$m, tout particulièrement de 14 à 30 $\mu$m

   * et d'eau (W) comme liant

   le rapport pondéral Rm de la quantité d'eau totale (WT) à la quantité de silice exprimée en sec étant supérieur à 1,4 et inférieur à 3, de préférence supérieur à 1,5 et inférieur à 3.

2. Granulés selon la revendication 1), **caractérisés en ce que** l'appareil de granulation est un appareil à rotor équipé de socs de charrue.

3. Granulés selon la revendication 1) ou 2), **caractérisés en ce que** l'appareil de granulation fonctionne en continu ou discontinu.

4. Granulés selon l'une quelconque des revendications 1) à 3), **caractérisés en ce que** le temps de granulation est de 10 à 45 minutes.

5. Granulés selon la revendication 4), **caractérisés en ce que** l'appareil de granulation développe en bout de rotor une vitesse périphérique d'au plus 4 m.s et **en ce que** le temps de granulation est de 20 à 45 minutes.

6. Granulés selon la revendication 4), **caractérisés en ce que** l'appareil de granulation développe en bout de rotor une vitesse périphérique supérieure à 4 m.s et **en ce que** le temps de granulation est de 10 à 20 minutes.

7. Granulés selon l'une quelconque des revendications 1) à 5), **caractérisés en ce que** l'appareil de granulation développe en bout de rotor une vitesse périphérique d'au plus 4 m.s et **en ce que** le rapport Rm est supérieur à 1,8 et inférieur à 2,5.

8. Granulés selon l'une quelconque des revendication 1) à 4) et 6), **caractérisés en ce que** l'appareil de granulation développe en bout de rotor une vitesse périphérique supérieure à 4 m.s et **en ce que** le rapport Rm est supérieur à 1,4 et inférieur à 3, de préférence supérieur à 1,5 et inférieur à 3.

9. Granulés selon l'une quelconque des revendications 1) à 8), **caractérisés en ce que** la poudre de silice présente un diamètre $D_{10}$ d'au moins 1 $\mu$m, de préférence d'au moins 3 $\mu$m, et inférieur à 10 $\mu$m.

**10.** Granulés selon l'une quelconque des revendications 1) à 9), **caractérisés en ce qu'**ils présentent un facteur de cohésion FC de 0,45 à 0,75, de préférence de 0,5 à 0,7.

**11.** Granulés selon la revendication 10), **caractérisés en ce qu'**ils présentent un diamètre médian de 300 à 450$\mu$m, de préférence de 300 à 400 $\mu$m.

**12.** Utilisation des granulés faisant l'objet de l'une quelconque des revendications 1) à 11), comme additifs dans les compositions pour le nettoyage et/ou le traitement de la peau, des dents et autres surfaces dures, à raison de 0,1 à 50% du poids desdites compositions.

**13.** Utilisation selon la revendication 12), comme agent exfoliant dans les compositions cosmétiques à raison de 0,5 à 5% du poids desdites compositions.

**14.** Utilisation selon la revendication 12), comme agent nettoyant dans les compositions de nettoyage des surfaces dures à raison de 20 à 50% du poids desdites compositions.

**15.** Utilisation selon la revendication 12), comme agent épaississant à effet sensoriel en bouche dans les compositions dentaires.

**16.** Compositions dentaires comprenant de 0,1 à 12%, de préférence de 0,5 à 10% en poids des granulés faisant l'objet des revendications 1) à 11).

**Claims**

**1.** High-structure (thickening) precipitation silica granules with a median diameter of at least 150 $\mu$m and preferably of at least 250 $\mu$m, obtained by wet granulation of high-structure (thickening) precipitation silica, drying and optionally screening,
said granules being **characterized in that** they have a cohesion factor CF of at least 0.3 and preferably from 0.4 to less than 0.9, and **in that** the granulation step is performed using

* a rotor machine comprising shear means capable of developing a peripheral speed at the end of the rotor ranging from 0.5 m.s to 8 m.s and preferably from 2 m.s to 6 m.s
* at least one high-structure (thickening) precipitation silica powder having

. a DOP oil uptake of at least 230 ml/100 g and preferably of at least 290 ml/100 g
. a median diameter $D_{50}$ of at least 14 $\mu$m, preferably from 14 to 80 $\mu$m and most particularly from 14 to 30 $\mu$m

* and water (W) as binder

the weight ratio Rm of the amount of total water (WT) to the amount of silica expressed as dry matter being greater than 1.4 and less than 3, preferably greater than 1.5 and less than 3.

**2.** The granules as claimed in claim 1, **characterized in that** the granulating machine is a plowshare rotor machine.

**3.** The granules as claimed in claim 1 or 2, **characterized in that** the granulating machine operates in continuous or batch mode.

**4.** The granules as claimed in any one of claims 1 to 3, **characterized in that** the granulating time is from 10 to 45 minutes.

**5.** The granules as claimed in claim 4, **characterized in that** the granulating machine develops at the end of the rotor a peripheral speed of up to 4 m.s and **in that** the granulating time is from 20 to 45 minutes.

**6.** The granules as claimed in claim 4, **characterized in that** the granulating machine develops at the end of the rotor a peripheral speed of greater than 4 m.s and **in that** the granulating time is from 10 to 20 minutes.

**7.** The granules as claimed in any one of claims 1 to 5, **characterized in that** the granulating machine develops at the end of the rotor a peripheral speed of up to 4 m.s and **in that** the ratio Rm is greater than 1.8 and less than 2.5.

8.  The granules as claimed in any one of claims 1 to 4 and 6, **characterized in that** the granulating machine develops at the end of the rotor a peripheral speed of greater than 4 m.s and **in that** the ratio Rm is greater than 1.4 and less than 3 and preferably greater than 1.5 and less than 3.

9.  The granules as claimed in any one of claims 1 to 8, **characterized in that** the silica powder has a diameter $D_{10}$ of at least 1 $\mu$m and preferably of at least 3 $\mu$m, and less than 10 $\mu$m.

10. The granules as claimed in any one of claims 1 to 9, **characterized in that** they have a cohesion factor CF of from 0.45 to 0.75 and preferably from 0.5 to 0.7.

11. The granules as claimed in claim 10, **characterized in that** they have a median diameter of from 300 to 450 $\mu$m and preferably from 300 to 400 $\mu$m.

12. The use of the granules forming the subject of any one of claims 1 to 11, as additives in compositions for cleansing and/or treating the skin, the teeth and other hard surfaces, in a proportion of from 0.1% to 50% of the weight of said compositions.

13. The use as claimed in claim 12, as an exfoliant in cosmetic compositions, in a proportion of from 0.5% to 5% of the weight of said compositions.

14. The use as claimed in claim 12, as a cleaning agent in compositions for cleaning hard surfaces, in a proportion of from 20% to 50% of the weight of said compositions.

15. The use as claimed in claim 12, as a thickener with a mouthfeel effect in dental compositions.

16. Dental compositions comprising from 0.1% to 12% and preferably from 0.5% to 10% by weight of the granules forming the subject of claims 1 to 11.


**Patentansprüche**

1.  Granulat hochstrukturierter Fällungskieselsäure (Verdickungsmittel), das einen medianen Durchmesser von mindestens 150 $\mu$m, vorzugsweise von mindestens 250 $\mu$m aufweist, das durch Granulation von hochstrukturierter Fällungskieselsäure (Verdickungsmittel) auf nasschemischem Weg, Trocknung und gegebenenfalls Sieben erhalten wird,
    wobei das Granulat **dadurch gekennzeichnet ist, dass** es einen Kohäsionsfaktor FC von mindestens 0,3, vorzugsweise von 0,4 bis weniger als 0,9 aufweist und dass der Schritt der Granulation umgesetzt wird durch Einsetzen

    * eines Rotationsapparats, der Schermittel umfasst, die in der Lage sind, eine Umfangsgeschwindigkeit am Rand des Rotors zu entwickeln, die von 0,5 m.s bis 8 m.s, vorzugsweise von 2 m.s bis 6 m.s reicht,
    * mindestens eines Pulvers hochstrukturierter Fällungskieselsäure (Verdickungsmittel), das

    . eine DOP-Ölaufnahme von mindestens 230 ml/100 g, vorzugsweise von mindestens 290 ml/100 g,
    . einen medianen Durchmesser $D_{50}$ von mindestens 14 $\mu$m, vorzugsweise von 14 bis 80 $\mu$m, insbesondere von 14 bis 30 $\mu$m,

    aufweist,
    * und von Wasser (W) als Bindemittel,

    wobei das Gewichtsverhältnis Rm der Gesamtmenge an Wasser (WT) zur Trockenmenge an Kieselsäure größer als 1,4 und kleiner als 3, vorzugsweise größer als 1,5 und kleiner als 3 ist.

2.  Granulat gemäß Anspruch 1, **dadurch gekennzeichnet, dass** der Granulierapparat ein Rotationsapparat ist, der mit Klingen ausgestattet ist.

3.  Granulat gemäß Anspruch 1 oder 2, **dadurch gekennzeichnet, dass** der Granulierapparat kontinuierlich oder diskontinuierlich arbeitet.

4. Granulat gemäß einem der Ansprüche 1 bis 3, **dadurch gekennzeichnet, dass** die Granulierdauer 10 bis 45 Minuten beträgt.

5. Granulat gemäß Anspruch 4, **dadurch gekennzeichnet, dass** der Granulierapparat am Rand des Rotors eine Umfangsgeschwindigkeit von höchstens 4 m.s entwickelt und dass die Granulierdauer 20 bis 45 Minuten beträgt.

6. Granulat gemäß Anspruch 4, **dadurch gekennzeichnet, dass** der Granulierapparat am Rand des Rotors eine Umfangsgeschwindigkeit von mehr als 4 m.s entwickelt und dass die Granulierdauer 10 bis 20 Minuten beträgt.

7. Granulat gemäß einem der Ansprüche 1 bis 5, **dadurch gekennzeichnet, dass** der Granulierapparat am Rand des Rotors eine Umfangsgeschwindigkeit von höchstens 4 m.s entwickelt und dass das Verhältnis Rm größer als 1,8 und kleiner als 2,5 ist.

8. Granulat gemäß einem der Ansprüche 1 bis 4 und 6, **dadurch gekennzeichnet, dass** der Granulierapparat am Rand des Rotors eine Umfangsgeschwindigkeit von mehr als 4 m.s entwickelt und dass das Verhältnis Rm größer als 1,4 und kleiner als 3, vorzugsweise größer als 1,5 und kleiner als 3 ist.

9. Granulat gemäß einem der Ansprüche 1 bis 8, **dadurch gekennzeichnet, dass** das Pulver der Kieselsäure einen Durchmesser $D_{10}$ von mindestens 1 $\mu$m, vorzugsweise von mindestens 3 $\mu$m und von weniger als 10 $\mu$m aufweist.

10. Granulat gemäß einem der Ansprüche 1 bis 9, **dadurch gekennzeichnet, dass** es einen Kohäsionsfaktor FC von 0,45 bis 0,75, vorzugsweise von 0,5 bis 0,7 aufweist.

11. Granulat gemäß Anspruch 10, **dadurch gekennzeichnet, dass** es einen medianen Durchmesser von 300 bis 450 $\mu$m, vorzugsweise von 300 bis 400 $\mu$m aufweist.

12. Verwendung des Granulats, das unter einen der Ansprüche 1 bis 11 fällt, als Additiv in Zusammensetzungen zur Reinigung und/oder zur Behandlung von Haut, Zähnen oder anderen harten Oberflächen, in einem Anteil von 0,1 bis 50 Gew.-% dieser Zusammensetzungen.

13. Verwendung gemäß Anspruch 12 als Peeling-Mittel in kosmetischen Zusammensetzungen in einem Anteil von 0,5 bis 5 Gew.-% dieser Zusammensetzungen.

14. Verwendung gemäß Anspruch 12 als Reinigungsmittel in Reinigungszusammensetzungen für harte Oberflächen in einem Anteil von 20 bis 50 Gew.-% dieser Zusammensetzungen.

15. Verwendung gemäß Anspruch 12 als Verdickungsmittel in Dentalzusammensetzungen mit spürbarer Wirkung im Mund.

16. Dentalzusammensetzungen, umfassend 0,1 bis 12 Gew.-%, vorzugsweise 0,5 bis 10 Gew.-% des Granulats, das unter die Ansprüche 1 bis 11 fällt.